# EUROPEAN PATENT SPECIFICATION

(11) **EP 4 507 587 B1**
(45) Date of publication and mention of the grant of the patent: **06.05.2026**
(21) Application number: 23722144.5
(22) Date of filing: 12.04.2023
(51) Int. Cl.: A61B 17/11, A61B 17/115, A61B 17/064, A61B 17/00

(54) **COMPRESSION ANASTOMOSIS RINGS WITH ADJUSTABLE COMPRESSION PROFILE**
ANASTOMOSE-KOMPRESSIONSRINGE MIT EINSTELLBAREM KOMPRESSIONSPROFIL
ANNEAUX D'ANASTOMOSE PAR COMPRESSION À PROFIL DE COMPRESSION AJUSTABLE

(30) Priority: 13.04.2022 US 202263330483 P
(43) Date of publication of application: 19.02.2025
(73) Proprietor: Covidien LP, Mansfield, MA 02048 (US)
(72) Inventor: CAULK, Alexander W., North Haven, Connecticut 06473 (US); NICHOLAS, David A., North Haven, Connecticut 06473 (US); STRASSNER, Haley E., North Haven, Connecticut 06473 (US); ESCHBACH, Matthew S., North Haven, Connecticut 06473 (US)
(74) Representative: Zimmermann & Partner Patentanwälte mbB
(86) International application number: PCT/US2023/018251
(87) International publication number: WO 2023/200827

(56) References cited:
- EP-A1- 2 316 353
- US-A- 4 957 499
- US-A1- 2008 015 617

## Description

### CROSS REFERENCE TO RELATED APPLICATIONS

This application claims the benefit of and priority to U.S. Provisional Patent Application Serial No. 63/330,483 filed on April 13, 2022.

### BACKGROUND

### 1. Technical Field

The present disclosure relates to surgical devices. More specifically, the present disclosure relates to electromechanical surgical systems for performing surgical procedures.

### 2. Background of Related Art

In the event that some portion of the alimentary tract is removed, and continuity needs to be restored with an anastomosis, surgeons currently use surgical staplers or handsewn sutures to create the anastomosis. However, such techniques may result in certain complications, e.g., malformed staples. Thus, there is a need for a surgical device configured to form anastomoses of the alimentary canal using alternative fastening methods.
US4957499A describes a surgical suturing instrument for establishing circular compression anastomoses in the organs of the digestive tract. EP2316353A1 describes a surgical fastening apparatus. US2008/015617A1 describes a compression anastomosis ring assembly for use in joining severed organ wall portions of a hollow organ.

### SUMMARY

The invention is defined by the appended independent claims. Optional features are set out in the appended dependent claims.

The present disclosure provides an anastomosis forming device that accounts for differences in tissue properties by adjusting the compression gap according to an internal feedback mechanism. In particular, a powered surgical anastomosis device is provided that is configured to clamp, compress, and lock a compression ring assembly to form an anastomosis. The compression ring assembly includes two opposing rings configured to connect two sections of an alimentary tract (e.g., intestine, colon, etc.) The powered surgical device includes a handle assembly having a power source and one or more motors coupled to the power source. The device also includes an adapter assembly having multiple transmission assemblies, e.g., drive shafts, which transmit actuation from the powered handle. The powered handle assembly and the adapter assembly may be reusable. The adapter assembly includes an end effector having an anvil and a reload configured to engage the compression ring assembly to move the two rings together. The end effector also includes compression and locking actuation mechanisms to secure the compression ring assembly to the alimentary tract thereby forming the anastomosis. The end effector also includes a cutting mechanism, i.e., an annular cutter, to restore the lumen of the alimentary tract.

The powered surgical device operates in four phases, namely, compressing, locking, cutting, and unclamping. Clamping is accomplished by moving the anvil in a proximal direction to compress tissue held within the compression ring assembly and/or moving a portion of the reload in a distal direction. During compression, the rings are further approximated until a desired compression pressure is reached. Locking is accomplished by securing the rings of the compression ring assembly at the compressed distance. The lumen of the tissue is restored by advancing a circular knife to cut tissue from the center of the rings during the cutting phase. During unclamping, the anvil is disengaged from the compression ring assembly and is retracted, allowing for removal of the powered surgical device from the alimentary tract.

The compression ring assembly includes a pair of opposing compression rings that are used to form the anastomosis. The compression rings may advantageously provide 1) more uniform distribution of pressure across tissue, 2) a reduced number of puncture sites to the tissue (i.e., few locking pins for the compression device vs. multiple staples), and 3) an adjustable compression gap based on real-time feedback indicators. In addition, the compression ring assembly also optimizes tissue compression pressure, and depending on data collected compression speed may be optimized for various tissue types and health conditions. Real-time feedback may include data from one or more sensors such as strain gauges for force, light absorption detectors for optical properties, bioimpedance sensors for electrical properties, or other similar sensors. Such feedback provides the opportunity to evaluate real-time changes to tissue properties during the compression phase of the anastomosis such that the compression gap may be prescribed by the system to optimize these target properties. This allows for tailoring of the compression based on different tissue loads for similar compression values. In addition, using the feedback during compression of the compression ring also reduces the chances of mechanical failure of the tissue since tissue properties, i.e., failure properties, depend upon compression. This is also an added advantage of the feedback loop and adjustable compression profile. In particular, optical and electrical properties change with tissue compression, allowing for sensing and compression optimization using various tissue properties which can inform clinically relevant metrics such as structural tissue damage, tissue perfusion, etc.

For device operation, the compression ring assembly, including two rings are inserted into the tissue of interest using the powered surgical device. A proximal ring is advanced toward a distal ring (i.e., the ring farther from the handle assembly) and/or a distal ring may be retracted toward the proximal ring using a motor and drive assembly until a feedback mechanism dictates that the compression is sufficient based on real-time feedback indicator (e.g., force), at which point the distal ring is fixed in place. Each of the anvil and the proximal ring may be advanced by their corresponding mechanisms, e.g., motors and transmission assemblies. A separate driver may then be advanced to move a lock ring to lock the rings with the appropriate gap and ensure proper fixation of the rings to the tissue.

In embodiments, the distal ring may remain stationary following calibration and the proximal ring is advanced distally to compress the tissue. A lock ring is then advanced with a separate motor. This two-step (i.e., compress and lock) approach ensures that the tissue compression force measurement is accurate and does not contain the force required to advance the lock ring. In further embodiments, the proximal ring may remain stationary and the distal ring is advanced proximally to compress tissue. The lock ring is similarly advanced to lock the rings in place. In yet further embodiments, both rings may be approximated to compress tissue, followed by moving the lock ring.

In embodiments, other locking mechanisms may be used, such as locking pins, which are advanced through both compression rings to lock the rings with the appropriate gap and ensure proper fixation of the rings to the tissue. Similar to currently available circular staplers, compression rings could be manufactured with a variety of diameters and materials, e.g., bioabsorbable, to accommodate different lumen sizes in the clinical population.

According to one embodiment of the present disclosure, a surgical device for forming an anastomosis is disclosed. The surgical device includes an anvil assembly having a head assembly coupled to a rod. The head assembly also includes an outer ring portion coupled to an inner portion via a connection portion. The device further includes an annular reload having a proximal ring with a plurality of pins movably disposed therein. The annular reload also includes a first driver movable distally and configured to engage the plurality of pins, an annular knife, and a second driver movable distally and configured to engage the annular knife. The device further includes a first transmission assembly coupled to and configured to move the anvil assembly proximally toward the annular reload to clamp tissue between the anvil assembly and the annular reload. The device also includes a second transmission assembly coupled to and configured to move the first driver distally to move the plurality of pins through the proximal ring, the tissue, and into the outer ring portion to embed the plurality of pins in the outer ring portion. The device may include a third transmission assembly coupled to and configured to move the second drive and the annular knife through the tissue and the connection portion of the head assembly to separate the outer ring portion from the inner portion.

Implementations of the above embodiment may include one or more of the following features. According to one aspect of the above embodiment, the anvil assembly may be formed from a thermoplastic polymer. The surgical device may include at least one motor configured to move at least one of the first transmission assembly, the second transmission assembly, or the third transmission assembly. The surgical device may also include a sensor configured to measure at least one property of the tissue. The sensor may be configured to measure at least one of a mechanical property, optical property, or electrical property of the tissue. The surgical device may also include a controller configured to determine whether an anastomosis has been formed or optimal conditions for forming an anastomosis based on at least one property. The controller may be further configured to control at least one motor to move the third transmission assembly based on the determination that the anastomosis has been formed thereby advancing the annular knife through the tissue and the connection portion of the head assembly to separate the outer ring portion from the inner portion.

According to one aspect of the present disclosure, a compression ring assembly is disclosed. The compression ring assembly includes a tubular support member and a first ring securely coupled to the tubular support member. The first ring is configured to engage a first segment of an alimentary tract portion. The compression ring assembly also includes a second ring slidably disposed on the tubular support member and movable along the tubular support member and relative to the first ring. The second ring is configured to engage a second segment of the alimentary tract portion. The first ring and the second ring are configured to be approximated relative to each other to compress the first segment and the second segment to form an anastomosis.

Implementations of the above embodiment may include one or more of the following features. According to one aspect of the above embodiment, the tubular support member may be configured to engage an anvil assembly. The tubular support member may include at least one protrusion configured to engage the anvil assembly. The compression ring assembly may also include a lock ring disposed on the tubular support member. The lock ring is movable along the tubular support member and relative to the second ring. The lock ring is configured to retain the second ring. The tubular support member may include a barbed surface configured to permit movement of the lock ring in a direction toward the second ring.

According to another aspect of the present disclosure, a system for forming an anastomosis is disclosed. The system includes a compression ring assembly having a tubular support member and a first ring securely coupled to the tubular support member. The first ring is configured to engage a first segment of an alimentary tract portion. The compression ring assembly also includes a second ring slidably disposed on the tubular support member and movable along the tubular support member and relative to the first ring. The second ring is configured to engage a second segment of the alimentary tract portion. The system also includes a powered surgical device having an annular reload configured to support the second ring. An anvil assembly is configured to engage the tubular support member. The first ring and the second ring are configured to be approximated relative to each other to compress the first segment and the second segment to form an anastomosis.

Implementations of the above embodiment may include one or more of the following features. According to one aspect of the above embodiment, the tubular support member may include at least one protrusion configured to engage the anvil assembly. The compression ring may also include a lock ring disposed on the tubular support member. The lock ring may be movable along the tubular support member and relative to the second ring. The lock ring may be configured to retain the second ring. The tubular support member may also include a barbed surface configured to permit movement of the lock ring in a direction toward the second ring. The annular reload may include a second driver longitudinally movable within the annular reload and configured to engage the lock ring. The powered surgical device may further include a first motor and a first transmission assembly coupled to the first motor and the anvil assembly, the first motor configured to move the anvil assembly. The annular reload may include a first driver longitudinally movable within the annular reload and configured to engage the second ring. The powered surgical device may further include a second motor and a second transmission assembly coupled to the second motor and the first driver. The second motor is configured to move the second ring. The powered surgical device may further include a third motor and a third transmission assembly coupled to the third motor and the second driver. The third motor is configured to move the lock ring.

According to a further aspect of the present disclosure, a system for forming an anastomosis is disclosed. The system includes a compression ring assembly having a tubular support member and a first ring securely coupled to the tubular support member. The first ring is configured to engage a first segment of an alimentary tract portion. The compression ring assembly also includes a second ring slidably disposed on the tubular support member and movable along the tubular support member and relative to the first ring. The second ring is configured to engage a second segment of the alimentary tract portion. The system also includes a powered surgical device having a controller, a motor, and a transmission assembly coupled to the motor. The powered surgical device also includes an annular reload configured to support the second ring and an anvil assembly configured to engage the tubular support member. The anvil assembly is movable by the transmission assembly and configured to move the tubular support member and the first ring. The first ring and the second ring are configured to be approximated relative to each other to compress the first segment and the second segment to form an anastomosis.

Implementations of the above embodiment may include one or more of the following features. According to one aspect of the above embodiment, the powered surgical device may include a sensor configured to measure strain imparted on the transmission assembly. The controller may be configured to determine whether the anastomosis has been formed or the optimal conditions for forming an anastomosis based on the measured strain. The tubular support member may include at least one protrusion configured to engage the anvil assembly. The compression ring may further include a lock ring disposed on the tubular support member. The lock ring may be movable along the tubular support member and relative to the second ring. The lock ring may be configured to retain the second ring. The tubular support member may include a barbed surface configured to permit movement of the lock ring in a direction toward the second ring.

### BRIEF DESCRIPTION OF THE DRAWINGS

Embodiments of the present disclosure are described herein with reference to the accompanying drawings, wherein:
FIG. 1 is a perspective view of a powered surgical device including a handle assembly, an adapter assembly, and an end effector, according to an embodiment of the present disclosure;
FIG. 2 is a perspective view of the end effector with a compression ring assembly (compression ring) assembly according to an embodiment of the present disclosure;
FIG. 3 is a perspective view of two separated sections of an alimentary tract portion with the end effector and the compression ring assembly being inserted to form an anastomosis;
FIG. 4 is a perspective, partially transparent view of the compression ring assembly joining two separated sections of the alimentary tract portion;
FIG. 5 is a perspective view of the compression ring assembly according to an embodiment of the present disclosure;
FIG. 6 is a cross-sectional view of the compression ring assembly;
FIG. 7 is a perspective view, with parts disassembled, of the compression ring assembly;
FIG. 8 is a schematic diagram of the handle assembly, the adapter assembly, and the end effector of FIG. 1;
FIG. 9 is a side perspective view of the adapter assembly and the end effector attached to the adapter assembly of FIG. 1 according to an embodiment of the present disclosure;
FIG. 10 is a perspective view of a first transmission assembly disposed within the adapter assembly of FIG. 1, shown partially in phantom;
FIG. 11 is a perspective view of a second transmission assembly disposed within the adapter assembly of FIG. 1, shown partially in phantom;
FIG. 12 is a perspective view of a third transmission assembly disposed within the adapter assembly of FIG. 1, shown partially in phantom;
FIG. 13 is a perspective view of an end effector according to an embodiment of the present disclosure;
FIG. 14 is a side-cross sectional view of the end effector with the compression ring assembly in an unclamped configuration according to an embodiment of the present disclosure;
FIG. 15 is a side-cross sectional view of the end effector with the compression ring assembly in a clamped configuration according to an embodiment of the present disclosure;
FIG. 16 is a perspective view of the adapter assembly, shown partially disassembled, with a strain gauge assembly;
FIG. 17 is a cross-sectional view of the end effector with the compression ring assembly in the unclamped configuration inside the alimentary tract portion according to an embodiment of the present disclosure;
FIG. 18 is a cross-sectional view of the end effector with the compression ring assembly in the clamped configuration inside the alimentary tract portion according to an embodiment of the present disclosure;
FIG. 19 is a cross-sectional view of the end effector with the compression ring assembly in a locked configuration inside the alimentary tract portion according to an embodiment of the present disclosure;
FIG. 20 is a perspective view the compression ring assembly joining two sections of the alimentary tract portion;
FIG. 21 is a side, cross-sectional view the compression ring assembly joining two sections of the alimentary tract portion;
FIG. 22 is a perspective view of the end effector and an anvil assembly decoupled from the compression ring assembly joining two sections of the alimentary tract portion;
FIG. 23 is a perspective view of an end effector including an anvil assembly and a reload for deploying a compression ring assembly according to another embodiment of the present disclosure;
FIG. 24 is a side, cross-sectional view of the anvil assembly separated from the reload of FIG. 23;
FIG. 25 is a perspective view with parts separated of the anvil assembly of FIG. 23;
FIG. 26 is a perspective view with parts separated of the reload of FIG. 23;
FIGS. 27A and B are front and rear perspective views of a proximal ring of the compression ring assembly of FIG. 23;
FIG. 28A is a perspective view of the anvil assembly in inside a first alimentary tract portion and the end effector in a second alimentary tract portion according to an embodiment of the present disclosure;
FIG. 28B is a perspective view of the anvil assembly coupled to the end effector joining two alimentary tract portions according to an embodiment of the present disclosure;
FIG. 29 is a side view of the end effector with the compression ring assembly in a clamped configuration inside the alimentary tract portion according to an embodiment of the present disclosure;
FIG. 30 is a side, cross-sectional view of the end effector with the compression ring assembly in a clamped configuration inside the alimentary tract portion according to an embodiment of the present disclosure;
FIG. 31 is a side, cross-sectional view of the end effector with the compression ring assembly in the clamped configuration and a deployed knife assembly inside the alimentary tract portion according to an embodiment of the present disclosure;
FIG. 32 is a side, cross-sectional view of the compression ring assembly in the clamped configuration inside the alimentary tract portion according to an embodiment of the present disclosure; and
FIG. 33 is a perspective view of the compression ring assembly in the clamped configuration inside the alimentary tract portion according to an embodiment of the present disclosure.

### DETAILED DESCRIPTION OF EMBODIMENTS

Embodiments of the presently disclosed surgical devices, and adapter assemblies for surgical devices and/or handle assemblies are described in detail with reference to the drawings, in which like reference numerals designate identical or corresponding elements in each of the several views. As used herein the term "distal" refers to that portion of the surgical instrument, or component thereof, farther from the user, while the term "proximal" refers to that portion of the surgical instrument, or component thereof, closer to the user.

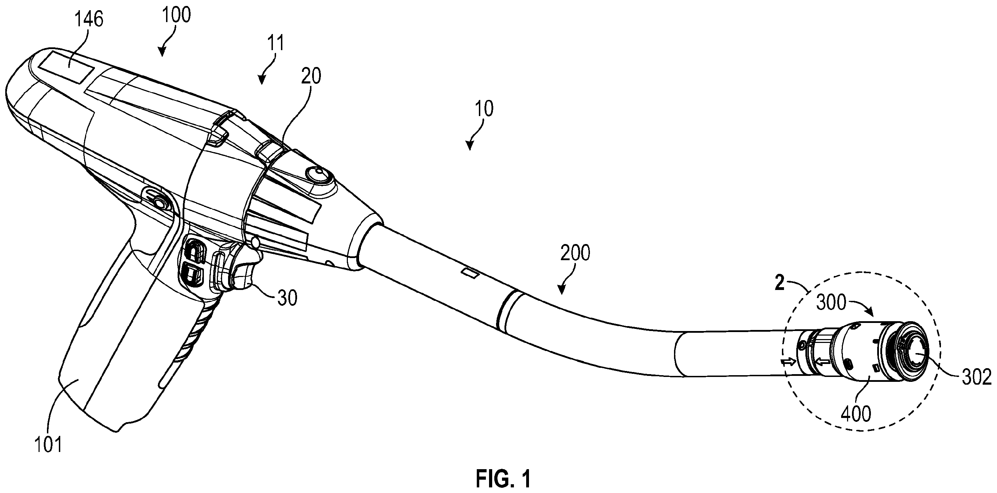

FIG. 1 illustrates a surgical device, such as, for example, a powered surgical device 10 for forming end-to-end anastomosis ("EEA"), including a handle assembly 100, which is configured for selective connection with an adapter assembly 200. The adapter assembly 200 is configured for selective connection with an end effector 300, which includes an annular reload 400 and an anvil assembly 302. The end effector 300 is configured to produce a surgical effect on tissue of a patient, namely, forming an anastomosis by connecting two portions of alimentary tract portion (e.g., intestine, colon, etc.).

The handle assembly 100 includes a power handle 101 and an outer shell housing 11 configured to selectively receive and encase power handle 101. The shell housing 11 includes a distal half-section 11a and a proximal half-section 11b pivotably connected to distal half-section 11a. When joined, distal and proximal half-sections 11a, 11b define a shell cavity therein in which power handle 101 is disposed.

While the powered surgical device 10 is described herein as a modular device including a plurality of interconnected components, such as the handle assembly 100, the removable shell housing 11, and the adapter assembly 200, etc. The powered surgical device 10 may be formed as an integrated device with one or more of the components being securely attached to each other, e.g., during manufacturing of the powered surgical device.

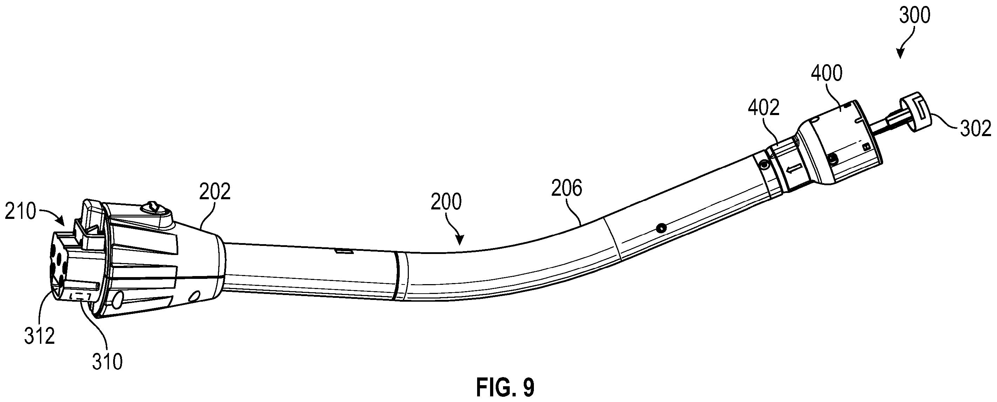

Distal and proximal half-sections 11a, 11b of shell housing 11 are divided along a plane that traverses a longitudinal axis of adapter assembly 200. Distal half-section 11a of shell housing 11 defines a connecting portion 20 configured to accept a corresponding drive coupling assembly 210 (FIG. 9) of adapter assembly 200. Distal half-section 11a of shell housing 11 supports a toggle control button 30. Toggle control button 30 is capable of being actuated in four directions (i.e., a left, right, up, and down).

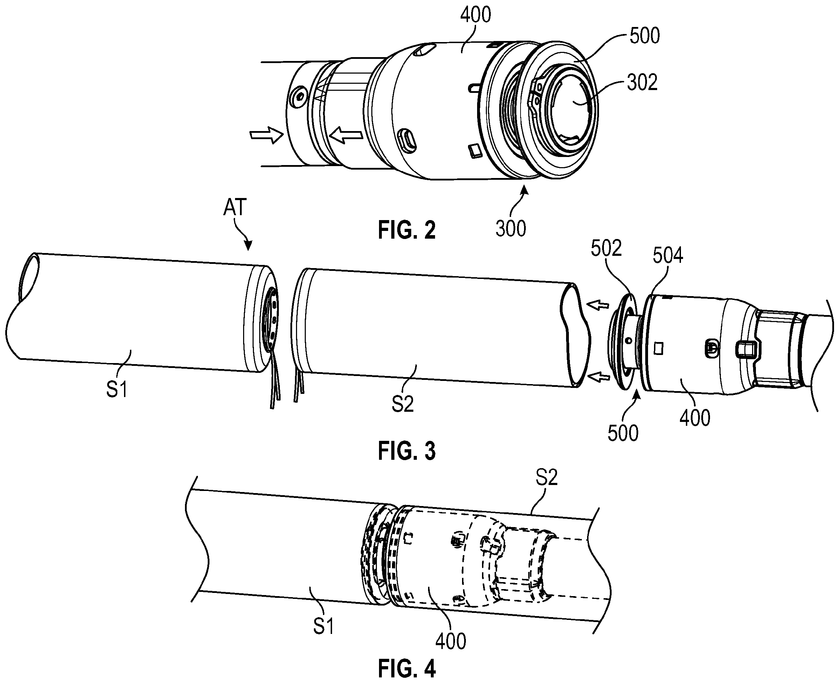

FIGS. 2-4 show a compression ring assembly 500 for forming an anastomosis between two sections S1 and S2 of an alimentary tract AT. The two sections S1 and S2 may be formed using any suitable surgical technique and may including previously formed purse sutures. The compression ring assembly 500 includes a distal ring 502 configured to engage an opening in the distal section S1 and a proximal ring 504 configured to engage an opening in the proximal section S2 of the alimentary tract AT. The distal and proximal rings 502 and 504 are approximated together to clamp and compress the tissue of the two sections S1 and S2 in the compression ring assembly 500 thereby forming an anastomosis.

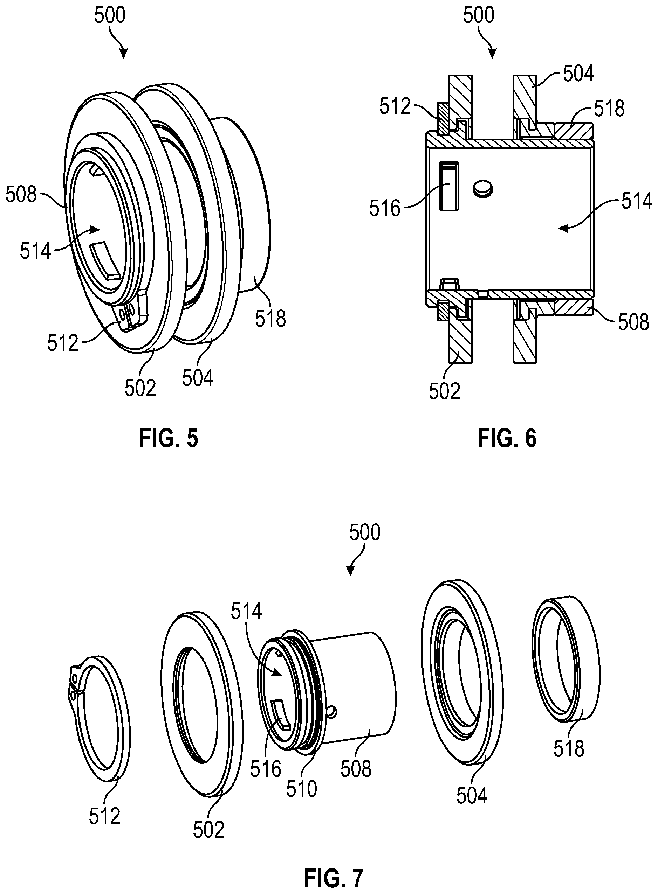

With reference to FIGS. 5-7, the compression ring assembly 500 includes a tubular support member 508 having a lip 510 for securing the distal ring 502 thereto. A retaining ring 512 may also be used to secure the distal ring 502 to the tubular support member 508. The tubular support member 508 defines lumen 514 allowing for the anvil assembly 302 to be inserted therein. In addition, one or more protrusions 516 are disposed within the lumen 514 and on an inner surface of the tubular support member 508. The protrusions 516 are configured to be engaged by the anvil assembly 302, such that as the anvil assembly 302 is moved longitudinally, the tubular support member 508 along with the distal ring 502 is moved correspondingly.

The proximal ring 504 of the compression ring assembly 500 is disposed on the tubular support member 508 and is configured to move slidingly on an outer surface of the tubular support member 508. A lock ring 518 is disposed proximally of the proximal ring 504 and is configured to move only in a distal direction thereby preventing proximal movement of the proximal ring 504, securing the compression ring assembly 500 in a clamped and locked configuration. Components of the compression ring assembly 500 may be formed from any suitable material including, but not limited to, metals, e.g., stainless steel, titanium, etc., bioabsorbable polymers or other suitable biocompatible thermoplastic polymers (e.g., polyether ether ketones).

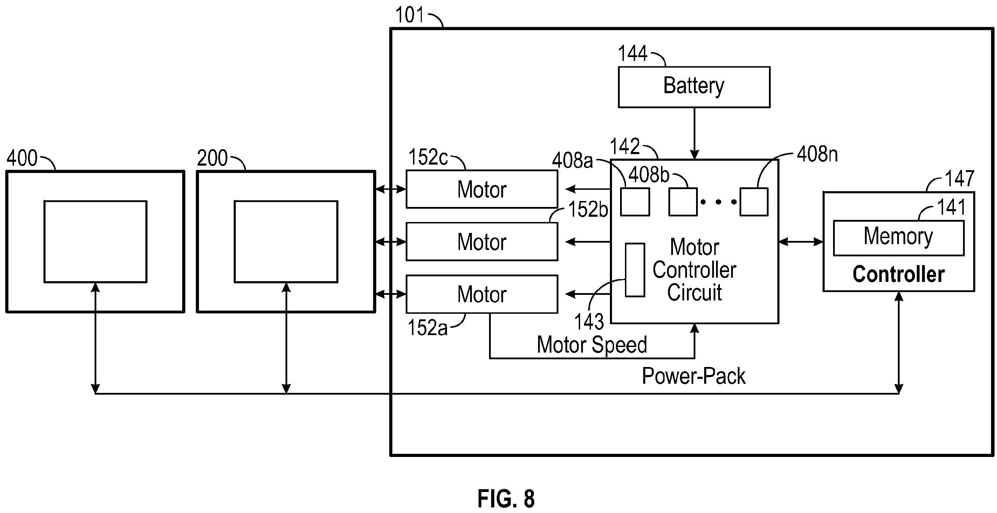

With reference to FIG. 8, the power handle 101 includes a main controller circuit board 142, a rechargeable battery 144 configured to supply power to any of the electrical components of handle assembly 100, and a plurality of motors, i.e., a first motor 152a, a second motor 152b, a third motor 152c coupled to the battery 144. The power handle 101 also includes a display 146. In embodiments, the motors 152a, 152b, 152c may be coupled to any suitable power source configured to provide electrical energy to the motors 152a, 152b, 152c, such as an AC/DC transformer. Each of the motors 152a, 152b, 152c is coupled to a motor controller 143 which controls the operation of the corresponding motors 152a, 152b, 152c including the flow of electrical energy from the battery 144 to the motors 152a, 152b, 152c. A main controller 147 is provided that controls the power handle 101. The main controller 147 is configured to execute software instructions embodying algorithms disclosed herein, such as clamping, compressing, locking, and cutting algorithms which control operation of the power handle 101.

The motor controller 143 includes a plurality of sensors 408a ... 408n, where "n" can be any number of sensors configured to measure operational states of the motors 152a, 152b, 152c and the battery 144. The sensors 408a-n include a strain gauge 408b and may also include voltage sensors, current sensors, temperature sensors, telemetry sensors, optical sensors, and combinations thereof. The sensors 408a-408n may measure voltage, current, and other electrical properties of the electrical energy supplied by the battery 144. The sensors 408a-408n may also measure angular velocity (e.g., rotational speed) as revolutions per minute (RPM), torque, temperature, current draw, and other operational properties of the motors 152a, 152b, 152c. The sensor 408a also includes an encoder configured to count revolutions or other indicators of the motors 152a, 152b, 152c, which is then use by the main controller 147 to calculate linear movement of components movable by the motors 152a, 152b, 152c. Angular velocity may be determined by measuring the rotation of the motors 152a, 152b, 152c or a drive shaft (not shown) coupled thereto and rotatable by the motors 152a, 152b, 152c. The position of various axially movable drive shafts may also be determined by using various linear sensors disposed in or in proximity to the shafts or extrapolated from the RPM measurements. In embodiments, torque may be calculated based on the regulated current draw of the motors 152a, 152b, 152c at a constant RPM. In further embodiments, the motor controller 143 and/or the main controller 147 may measure time and process the above-described values as a function of time, including integration and/or differentiation, e.g., to determine the rate of change in the measured values. The main controller 147 is also configured to determine distance traveled of various components of the adapter assembly 200 and/or the end effector 300 by counting revolutions of the motors 152a, 152b, 152c.

The motor controller 143 is coupled to the main controller 147, which includes a plurality of inputs and outputs for interfacing with the motor controller 143. In particular, the main controller 147 receives measured sensor signals from the motor controller 143 regarding operational status of the motors 152a, 152b, 152c and the battery 144 and, in turn, outputs control signals to the motor controller 143 to control the operation of the motors 152a, 152b, 152c based on the sensor readings and specific algorithm instructions. The main controller 147 is also configured to accept a plurality of user inputs from a user interface (e.g., switches, buttons, touch screen, etc. coupled to the main controller 147). The main controller 147 is also configured to receive optical and electrical sensor signals from sensors embedded in the reload 400.

The main controller 147 is also coupled to a memory 141. The memory 141 may include volatile (e.g., RAM) and non-volatile storage configured to store data, including software instructions for operating the power handle 101. The main controller 147 is also coupled to the strain gauge 408b of the adapter assembly 200 using a wired or a wireless connection and is configured to receive strain measurements from the strain gauge 408b which are used during operation of the power handle 101.

Turning now to FIG. 9, adapter assembly 200 includes an outer knob housing 202 and an outer tube 206 extending from a distal end of knob housing 202. Knob housing 202 and outer tube 206 are configured and dimensioned to house the components of adapter assembly 200. The knob housing 202 includes an electrical connector 312 and a storage device 310 coupled thereto. The storage device 310 is configured to store various operating parameters pertaining to the adapter assembly 200. Adapter assembly 200 is configured to convert rotation of coupling shafts (not explicitly shown) of handle assembly 100 into axial translations useful for operating the end effector 300.

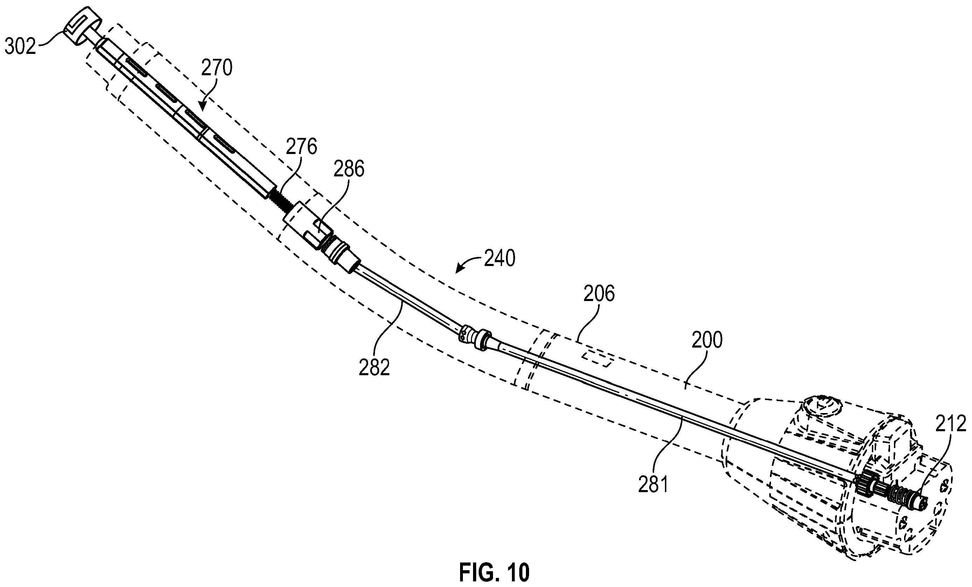

With reference to FIG. 10, adapter assembly 200 further includes the anvil coupler 270 removably supported in a distal end of outer tube 206. The anvil coupler 270 is axially and rotationally fixed within outer tube 206 of adapter assembly 200. Anvil coupler 270 is configured to couple a drive screw 276 to the anvil assembly 302, such that axial movement of anvil coupler 270, via a rotation of drive screw 276, results in a concomitant axial movement of anvil assembly 302.

A first transmission assembly 240 includes first rotatable proximal drive shaft 212 coupled to the first motor 152a, a second rotatable proximal drive shaft 281, a rotatable distal drive shaft 282, and a coupling member 286, each of which are supported within the outer tube 206 of adapter assembly 200. First transmission assembly 240 functions to extend/retract anvil assembly 302 of adapter assembly 200, and to retract the distal ring 502 of the compression ring assembly 500 when anvil assembly 302 is engaged with the tubular support member 508.

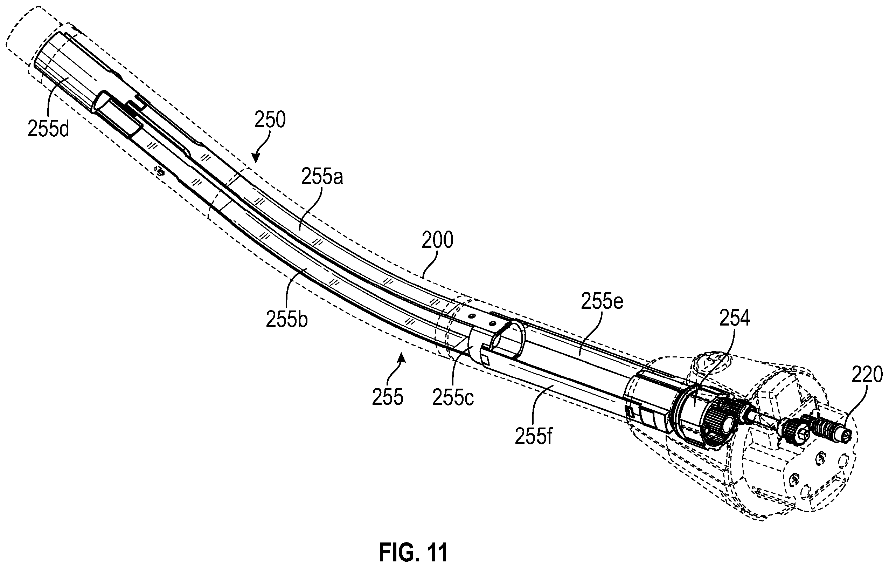

With reference to FIG. 11, the adapter assembly 200 includes a second transmission assembly 250 for interconnecting the second motor 152b and a first driver 430 of reload 400, wherein the second transmission assembly 250 converts and transmits a rotation of the second motor 152b to an axial translation of an outer flexible band assembly 255 of adapter assembly 200, and in turn, to move the first driver 430 of the reload 400 to move the proximal ring 504 in a distal axial direction.

The second transmission assembly 250 of adapter assembly 200 includes the outer flexible band assembly 255 secured to driver coupler 254. A second rotatable proximal drive shaft 220 is coupled to the second motor 152b and is configured to actuate that driver coupler 254, which converts rotational movement into longitudinal movement. Outer flexible band assembly 255 includes first and second flexible bands 255a, 255b laterally spaced and connected at proximal ends thereof to a support ring 255c and at distal ends thereof to a proximal end of a distal pusher 255d. Each of the first and second flexible bands 255a, 255b is attached to support ring 255c and distal pusher 255d. Outer flexible band assembly 255 further includes first and second connection extensions 255e, 255f extending proximally from support ring 255c. First and second connection extensions 255e, 255f are configured to operably connect outer flexible band assembly 255 to driver coupler 254 of second transmission assembly 250.

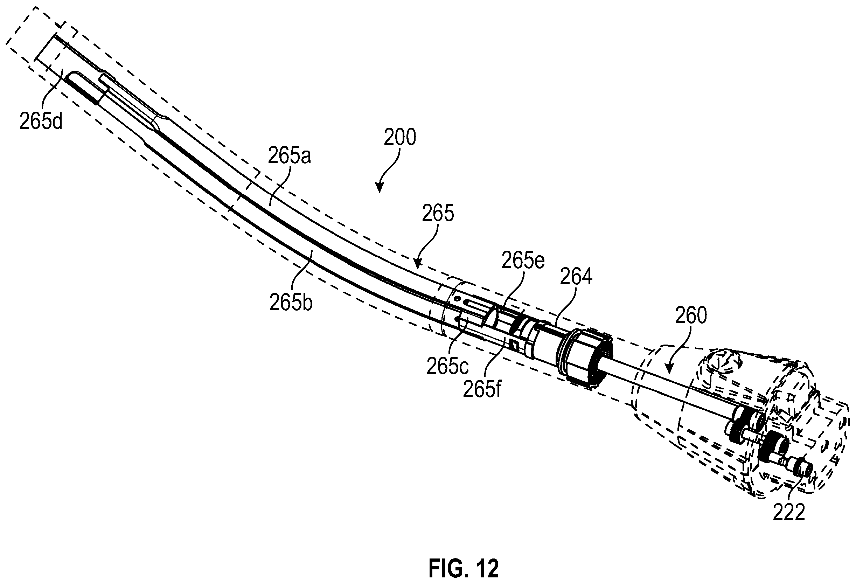

With reference to FIG. 12, the adapter assembly 200 also includes a third transmission assembly 260 having a third rotatable proximal drive shaft 222 for interconnecting the third motor 152c and a second driver 440 of reload 400, wherein the third transmission assembly 260 converts and transmits a rotation of the third motor 152c to an axial translation of an outer flexible band assembly 265 of adapter assembly 200, and in turn, to advance the second driver 440 of the reload 400 to move the lock ring 518.

Inner flexible band assembly 265 includes first and second flexible bands 265a, 265b laterally spaced and connected at proximal ends thereof to a support ring 265c and at distal ends thereof to a proximal end of a support base 265d. Each of first and second flexible bands 265a, 265b are attached to support ring 265c and support base 265d. Inner flexible band assembly 265 further includes first and second connection extensions 265e, 265f extending proximally from support ring 265c. First and second connection extensions 265e, 265f are configured to operably connect inner flexible band assembly 265 to driver 264 of third transmission assembly 260. Support base 265d extends distally from flexible bands 265a, 265b and is configured to connect with a second driver 440 of reload 400.

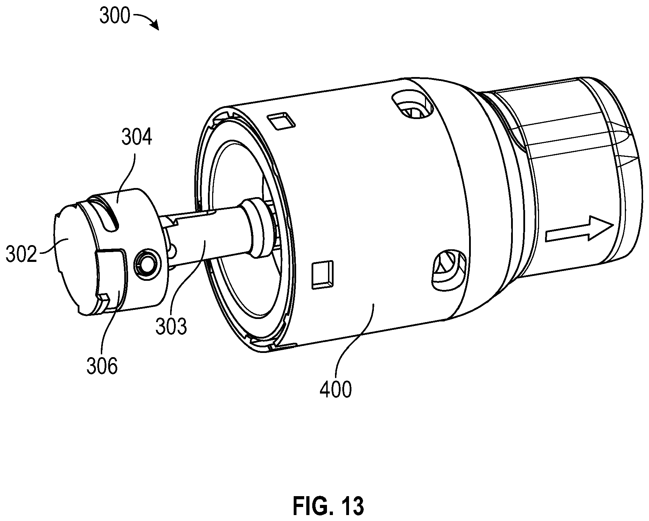

With reference to FIGS. 13-15, the anvil assembly 302 includes an anvil head 304 having one or more grooves or slots 306 that are configured to engage the protrusions 516 in a bayonet connection manner. This allows for securing of the tubular support member 508 to the anvil head 304. The anvil assembly 302 also includes a shaft 303 configured to interconnect the anvil assembly 302 to the anvil coupler 270. Axial movement of anvil coupler 270, via a rotation of drive screw 276, results in a concomitant axial movement of anvil assembly 302, which in turn moves the anvil head 304 along with the tubular support member 508 and the distal ring 502.

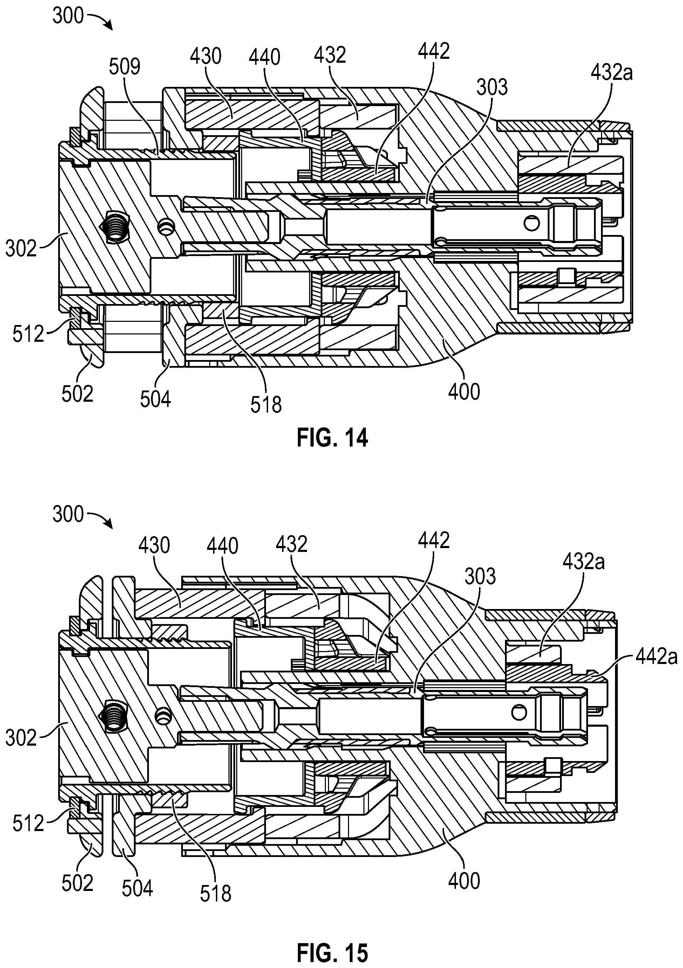

With reference to FIGS. 14 and 15, the tubular support member 508 is configured to be inserted into the reload 400 and the shaft 303 to be movable through the tubular support member 508. The reload 400 includes a first driver adapter 432 engaging the first driver 430. A proximal end 432a of the first driver adapter 432 is configured for selective contact and abutment with distal pusher 255d of outer flexible band assembly 255 of second transmission assembly 250 of adapter assembly 200. In operation, during distal advancement of outer flexible band assembly 255, as described above, distal pusher 255d of outer flexible band assembly 255 contacts proximal end 432a of the first driver adapter 432 to advance the first driver adapter 432 and first driver 430 from a first or proximal position to a second or distal position. Advancement of first driver 430 moves proximal ring 504 in the distal axial direction.

The reload 400 also includes a second driver adapter 442 engaging the second driver 440. A proximal end 442a of second driver adapter 442 is configured to engage the support base 265d of inner flexible band assembly. In operation, during distal advancement of inner flexible band assembly 265, support base 265d of inner flexible band assembly 265 connects with proximal end 442a of second driver adapter 442 to advance second driver adapter 442 and second driver 440 from a first or proximal position to a second or advanced position to move the lock ring 518 into a locking position. The tubular support member 508 also includes a barbed surface 509 configured to engage the lock ring 518 and prevent proximal movement thereof to secure the lock ring 518 once the lock ring 518 is advanced distally.

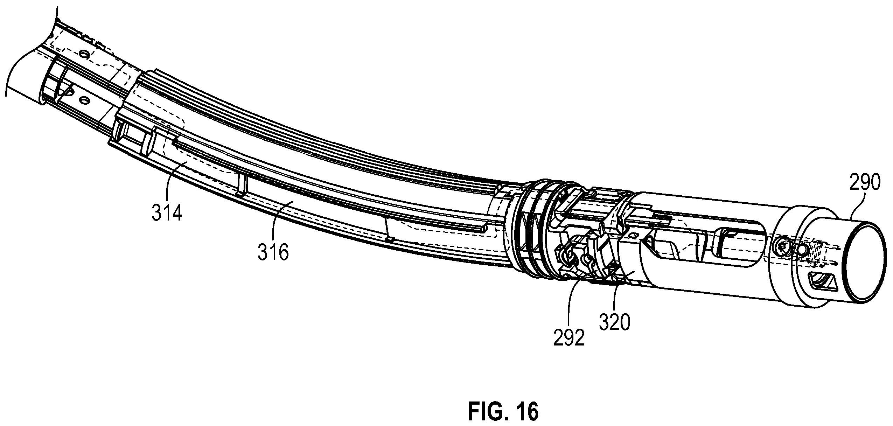

Forces during an actuation of compression ring assembly 500 may be measured by the strain gauge 408b in order to monitor and control various processes, such as clamping, compression, and locking of the compression ring assembly 500. With reference to FIG. 16, the strain gauge 408b of adapter assembly 200 is disposed within a strain gauge housing 320. In particular, the adapter assembly 200 includes a support block 292 fixedly disposed within outer tube 206. The strain gauge housing 320 is disposed between the support block 292 and a connector sleeve 290. The reload 400 is removably coupled to the connector sleeve 290.

In operation, strain gauge 408b of adapter assembly 200 measures and monitors the retraction of anvil assembly 302, since the anvil coupler 270 passes through the strain gauge 408b. The strain gauge 408b of adapter assembly 200 also measures and monitors movement of the proximal ring 504 and the lock ring 518, since the first and second flexible bands 255a, 255b also pass through the strain gauge 408b. During clamping, compression, and locking, a reaction force is exerted on anvil assembly 302 and the reload 400, which is communicated to support block 292, which then communicates the reaction force to a strain sensor of the strain gauge 408b.

Strain sensor of strain gauge 408b may be any device configured to measure strain (a dimensionless quantity) on an object that it is adhered to (e.g., support block 292), such that, as the object deforms, a metallic foil of the strain sensor is also deformed, causing an electrical resistance thereof to change, which change in resistance is then used to calculate loads experienced by anvil coupler 270.

The strain gauge 408b measures and monitors the retraction of anvil assembly 302 as well as movement of the proximal ring 504 and the lock ring 518. This distally directed reaction force is communicated from anvil assembly 302 to the strain gauge 408b. Strain gauge 408b is also electrically connected to the electrical connector 312 (FIG. 9) via proximal and distal harness assemblies 314, 316. The strain gauge 408b then communicates signals to main controller circuit board 142 of power handle 101 of handle assembly 100. This information may then be displayed on display 146 of handle assembly 100 to provide the user with real-time information related to the status of the handle assembly 100.

The reload 400 includes a storage device 402 and the circular adapter assembly 200 also includes a storage device 310 (FIG. 9). The storage devices 402 and 310 include non-volatile storage medium (e.g., EEPROM) that is configured to store any data pertaining to the reload 400 and the circular adapter assembly 200, respectively, including but not limited to, usage count, identification information, model number, serial number, stroke length, maximum actuation force, minimum actuation force, factory calibration data, and the like. In embodiments, the data may be encrypted and is only decryptable by devices (e.g., main controller 147) having appropriate keys. The data may also be used by the main controller 147 to authenticate the circular adapter assembly 200 and/or the reload 400. The storage devices 402 and 310 may be configured in read only or read/write modes, allowing the main controller 147 to read as well as write data onto the storage device 402 and 310.

Prior to operation of the powered surgical device 10, the power handle 101 is enclosed within the shell housing 11 and the adapter assembly 200 is coupled to handle assembly 100. After attachment of circular adapter assembly 200, handle assembly 100 initially verifies that circular adapter assembly 200 is coupled thereto by establishing communications with the storage device 310 of the circular adapter assembly 200 and authenticates circular adapter assembly 200. The data (e.g., usage count) stored on the storage device 310 is encrypted and is authenticated by the power handle 101 prior to determining whether the usage count stored on the storage device 310 exceeds the threshold (e.g., if the adapter assembly 200 has been previously used). Power handle 101 then performs verification checks (e.g., end of life checks, anvil assembly 302 missing, etc.) and calibrates circular adapter assembly 200 after the handle assembly 100 confirms that the anvil assembly 302 is attached.

The user commences a surgical procedure by attaching the compression ring assembly 500 to the anvil assembly 302. This may be done by aligning the protrusions 516 with the slots 306 and turning the anvil assembly 302 and/or the compression ring assembly 500. The anvil assembly 302 may be calibrated against a spacer to set a predetermined gap (e.g., approximately 6 mm) to accept the tissue.

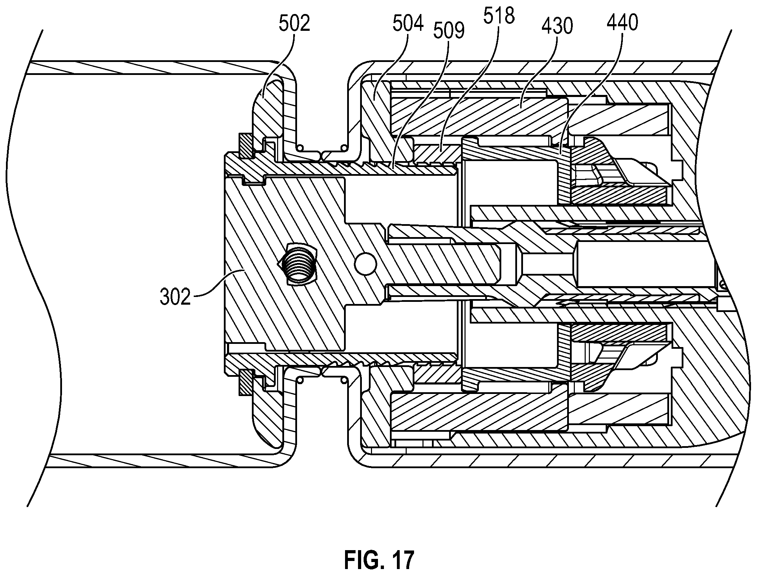

Thereafter, the user inserts the adapter assembly 200, including the anvil assembly 302 and the compression ring assembly 500, within the alimentary tract AT as shown in FIGS. 3 and 4, to ensure that the ends of the sections S1 and S2 of the alimentary tract AT are disposed between the distal and proximal rings 502 and 504 of the compression ring assembly 500 as shown in FIG. 17. The user presses the toggle control button 30 to begin the clamping process on the tissue interposed between the distal and proximal rings 502 and 504 by pressing on the bottom portion of the toggle control button 30.

The clamping process may be accomplished by approximating the distal and proximal rings 502 and 504 relative to each other, either both or one of the rings 502 and 504. In one embodiment, the anvil assembly 302 may remain stationary, while the proximal ring 504 is advanced distally by the first driver 430 to clamp tissue against the distal ring 502. In another embodiment, the proximal ring 504 may remain stationary, while the anvil assembly 302 is retracted, thereby moving the distal ring 502 proximally and clamping the tissue. In further embodiment, both of the distal and proximal rings 502 and 504 may be moved toward each other, either simultaneously or in a sequential manner to compress tissue.

The clamping process may continue until a clamping threshold is reached, after which, the process may transition to controlled tissue compression until a compression threshold is reached. In addition to monitoring the clamping threshold through the strain gauge 408b, clamping may be also monitored using light absorption detectors for monitoring optical properties of the tissue and/or bioimpedance sensors for monitoring electrical properties of the tissue. The detectors may be incorporated into the proximal ring 504 and/or 904. In further embodiments, clamping may be performed until the calibrated gap is reached. The thresholds and the gap may be adjustable during the compression process based on the feedback from the sensors.

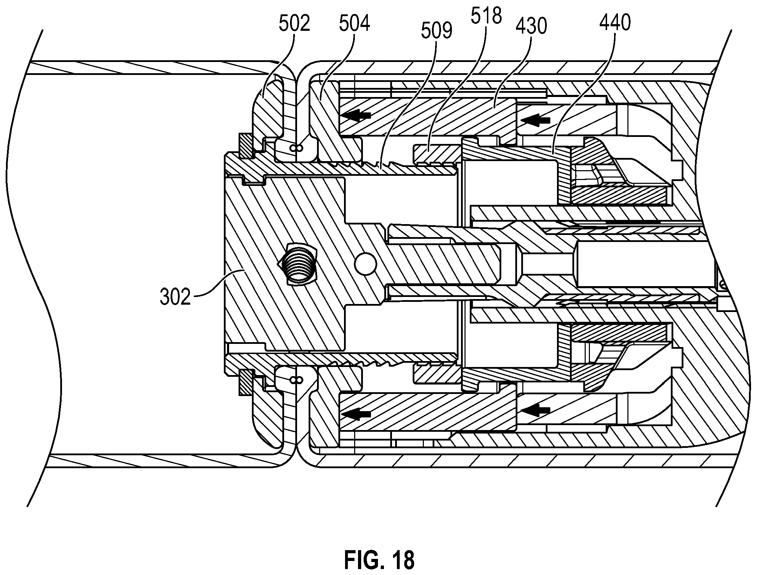

Compression of the tissue may include moving either both or one of the distal and proximal rings 502 and 504. In one embodiment, compression includes advancing the proximal ring 504 distally to further compress the clamped tissue as shown in FIG. 18. In another embodiment, compression may be achieved by retracting one or both of the distal ring 502 and the proximal ring 504.

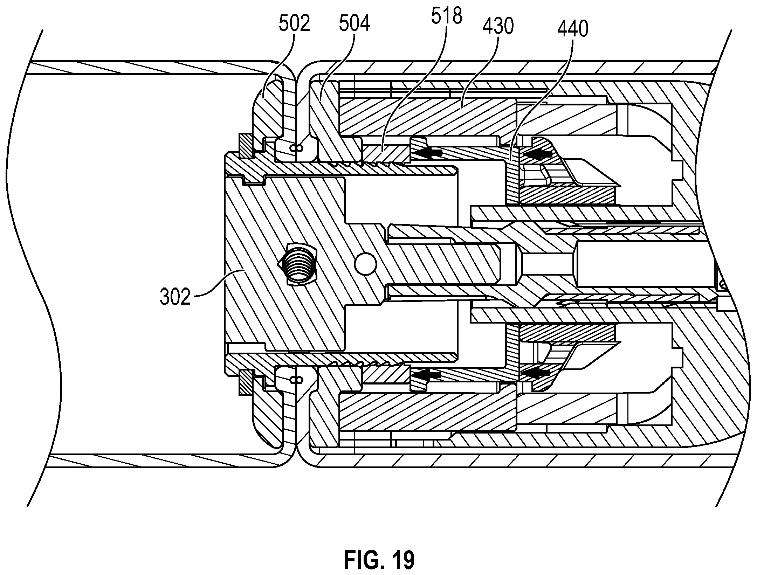

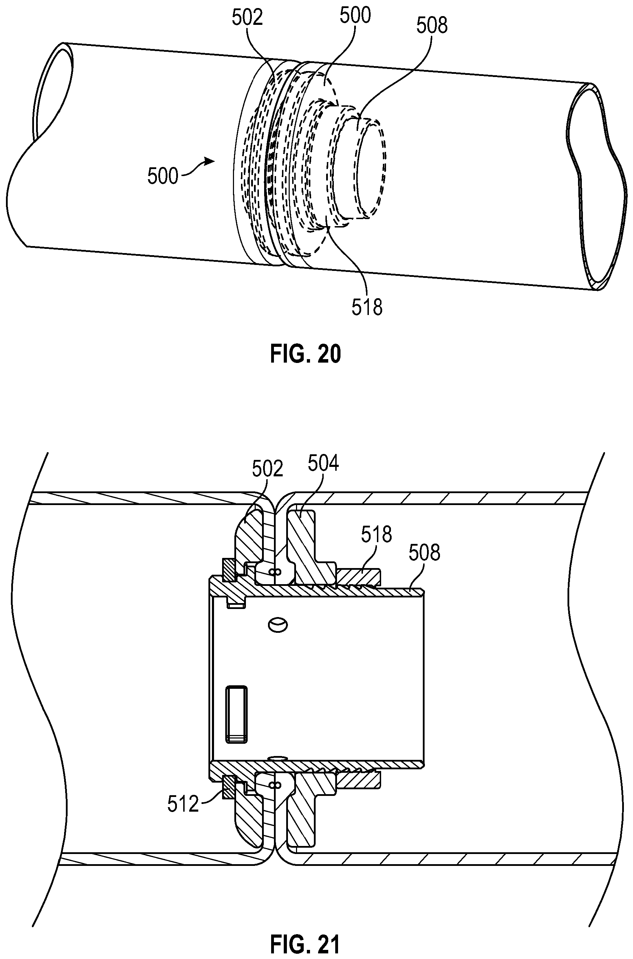

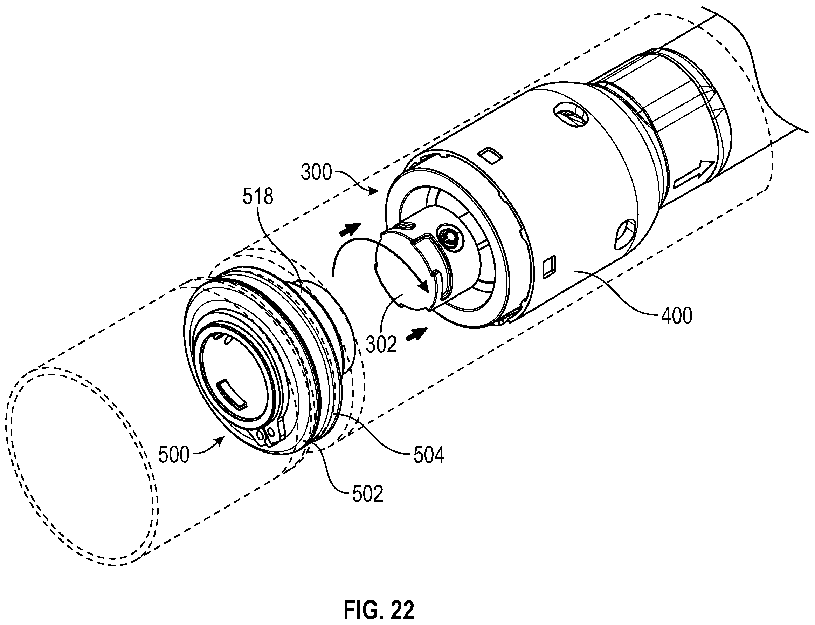

Once tissue is compressed, the locking process may be initiated manually by pressing the toggle control button 30 or automatically once tissue compression is confirmed by the main controller 147, i.e., compression threshold is reached. The locking process includes advancing the lock ring 518 along the tubular support member 508 until a locking force threshold is reached that is indicative of the lock ring 518 abutting the proximal ring 504 as shown in FIG. 19. The lock ring 518 is secured to the tubular support member 508 upon reaching the barbed surface 509. This secures the two sections S1 and S2 of the alimentary tract AT within the compression ring assembly 500 as shown in FIGS. 20 and 21. The anvil assembly 302 is then decoupled from the compression ring assembly 500 by twisting and retracting the anvil assembly 302 and/or the adapter assembly 200 as shown in FIG. 22.

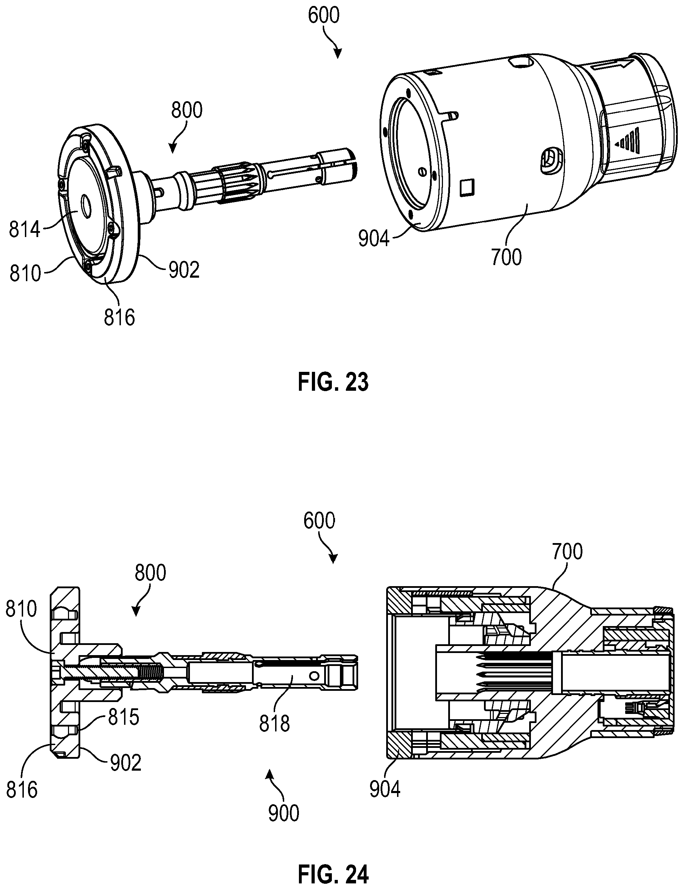

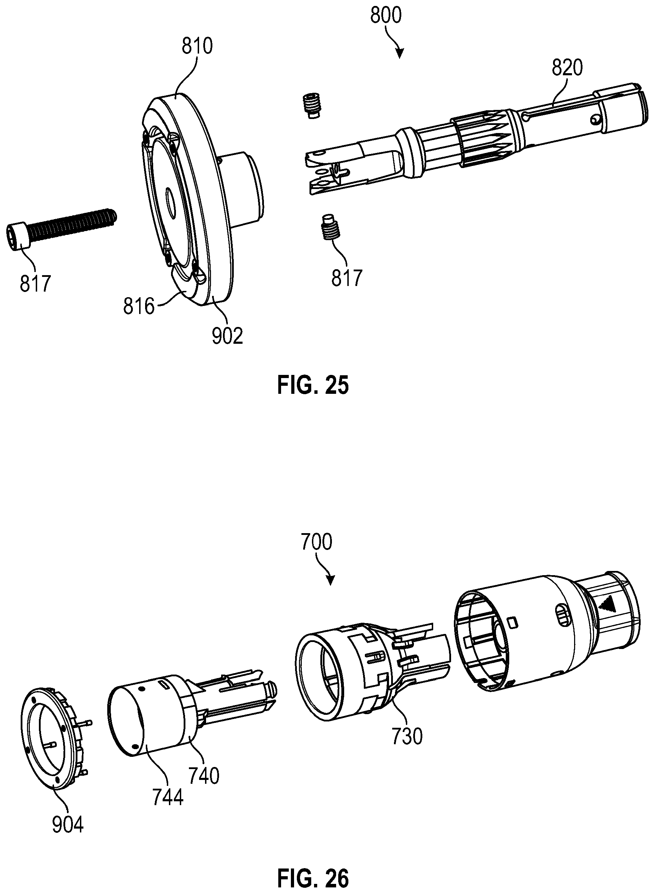

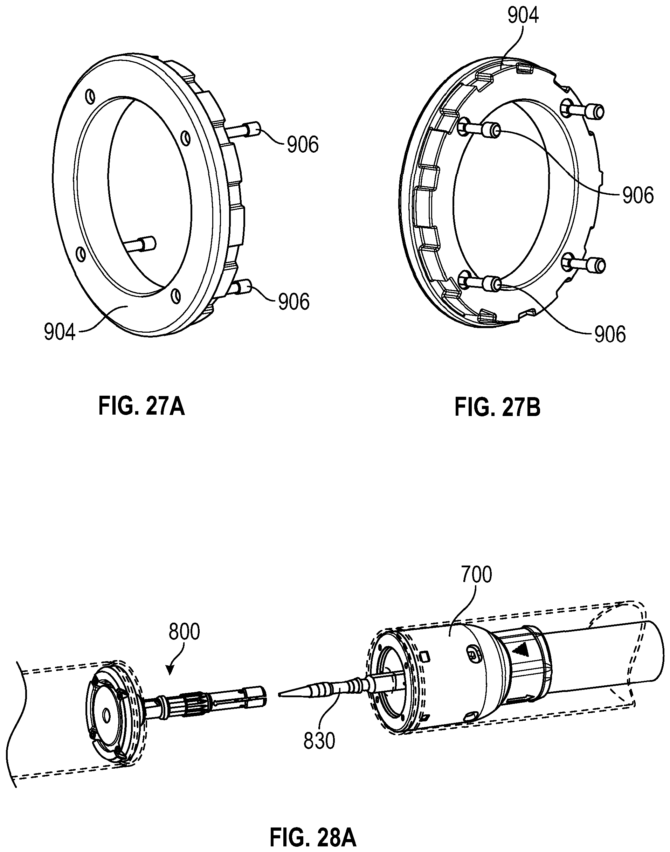

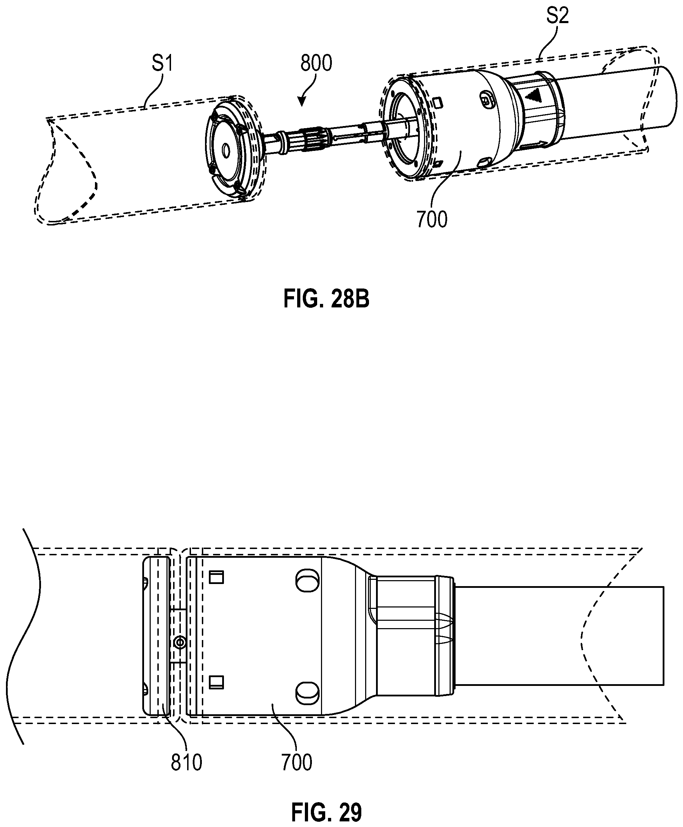

With reference to FIGS. 23-33, another embodiment of an end effector 600, which includes an annular reload 700 and an anvil assembly 800 is shown. As shown in FIGS. 23-25, the anvil assembly 800 includes a head assembly 810 secured to a rod 820. The head assembly 810 may be coupled to the rod 820 via one or more bolts 817 or any other suitable attachment mechanisms. The rod 820 includes a centrally defined lumen 818 configured to receive a trocar 830 (FIG. 28), which is coupled to the coupling member 286 of the first transmission assembly 240 (FIG. 10). The trocar 830 is configured to be inserted into the rod 820, such that axial movement of trocar 830, via the first transmission assembly 240, results in a concomitant axial movement of anvil assembly 800.

The end effector 600 houses a compression ring assembly 900, which includes a distal ring 902 forming an outer portion 816 of the head assembly 810 and a proximal ring 904 disposed in the reload 700. The head assembly 810 also includes an inner portion 814, which is attached to the rod 820, as described above. The outer portion 816 (i.e., the distal ring 902) is separably coupled, e.g., via cutting, to the inner portion 814 of the head assembly 810. The head assembly 810 may be formed from any material that may be cut to detach the outer portion 816 from the inner portion 814. Suitable materials for the head assembly 810 may be any thermoplastic polymer. The inner portion 814 is connected to the outer portion 816 via a connection portion 815 which is thinner than the inner portion 814 and the outer portion 816. The inner portion 814 is coupled to the rod 820 as described above, such that after separating the outer portion 816, the inner portion 814 remains attached to the rod 820, allowing for its withdrawal.

As shown in FIGS. 23, 24, and 26, the annular reload 700 is removably coupled to the connector sleeve 290. The reload 700 includes a first driver 730, which is configured to engage the distal pusher 255d of the second transmission assembly 250. With reference to FIG. 11, the second transmission assembly 250 interconnects the second motor 152b and the first driver 730 of reload 700, wherein the second transmission assembly 250 converts and transmits a rotation of the second motor 152b to an axial translation of an outer flexible band assembly 255 of adapter assembly 200, and in turn, to move the first driver 730 of the reload 700 to move distally and engage the proximal ring 904 disposed in the reload 700.

The reload 700 also includes a second driver 740, which is configured to engage the support base 265d of the third transmission assembly 260. With reference to FIG. 12, the third transmission assembly 260 interconnects the third motor 152c and the second driver 740 of reload 700, wherein the third transmission assembly 260 converts and transmits a rotation of the third motor 152c to an axial translation of an outer flexible band assembly 265 of adapter assembly 200, and in turn, to move the second driver 740 of the reload 700 to move distally and to move an annular knife 744 disposed concentrically within the proximal ring 904.

With reference to FIGS. 23, 24, 26, 27A-B, the proximal ring 904 includes a plurality (e.g., 2 or more) of locking pins 906. The proximal ring 904 includes a plurality of proximal openings configured to securely hold the locking pins 906, such that the locking pins 906 are movable by the first driver 730. The locking pins 906 may include an engagement surface configured abut the first driver 730.

With reference to FIGS. 28-31, forming an end-to-end anastomosis between separated alimentary tract portions S1 and S2 using the compression ring assembly 900 includes inserting and attaching the anvil assembly 800 to a distal portion S1 of the alimentary tract AT. The reload 700 while attached to the adapter assembly 200 is inserted into a proximal portion S2 of the alimentary tract AT. The trocar 830 is then inserted into the rod 820 to attach the anvil assembly 800. The user presses the toggle control button 30 to begin the clamping process on the tissue interposed between the distal and proximal rings 902 and 904 by pressing on the bottom portion of the toggle control button 30.

The clamping process may be accomplished by approximating the distal and proximal rings 902 and 904 relative to each other. The anvil assembly 800 is retracted by the first transmission assembly 240, thereby moving the distal ring 902 proximally and clamping the tissue. The clamping process may continue until a clamping threshold is reached, after which, the process may transition to controlled tissue compression until a compression threshold is reached. In addition to monitoring the clamping threshold through the strain gauge 408b, clamping may be also monitored using light absorption detectors for monitoring optical properties of the tissue and/or bioimpedance sensors for monitoring electrical properties of the tissue. In further embodiments, clamping may be performed until the calibrated gap is reached. The thresholds and the gap may be adjustable during the compression process based on the feedback from the sensors. Compression of the tissue may follow clamping by continued retraction of the anvil assembly 800 until a desired tissue compression is reached, which may also be determined based on measured strain through the strain gauge 408b. The powered surgical device 10 may indicate (e.g., on the display 146) that clamping and/or compression is complete.

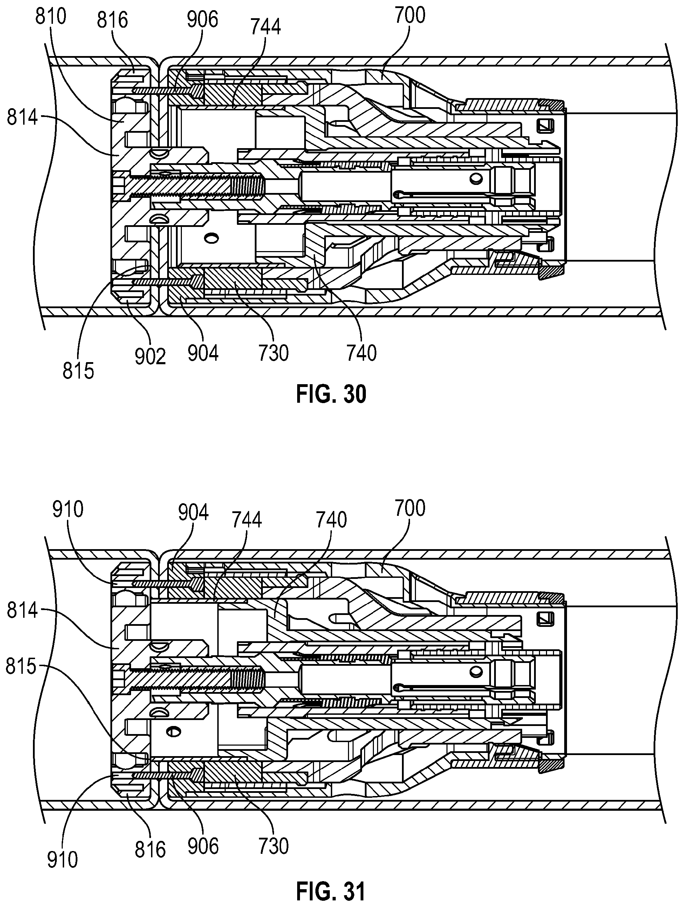

Once tissue is compressed, the locking process may be initiated manually, by pressing the toggle control button 30, or automatically once tissue compression is confirmed by the main controller 147, i.e., compression threshold is reached. The locking process includes advancing the first driver 730 distally, which pushes the locking pins 906 through the proximal ring 904 and into the distal ring 902. More specifically, the locking pins 906 may be aligned with a plurality of corresponding distal openings 910, which are configured to receive and retain the locking pins 906 therein. The alignment of the distal openings 910 may be accomplished by aligning the anvil assembly 800 to the trocar 830 via splines or other mechanical features disposed on the rod 820. The locking pins 906 may include barbs, ridges, or other unidirectional features to allow for the locking pins 906 to be secured within the distal openings 910, thereby securing the distal and proximal rings 902 and 904 once the locking pins 906 are embedded inside the distal ring 902. The locking pins 906 may be advanced until a locking force threshold is reached. The powered surgical device 10 may indicate (e.g., on the display 146) that locking is complete. This secures the two sections S1 and S2 of the alimentary tract AT within the compression ring assembly 900 as shown in FIGS. 29 and 30.

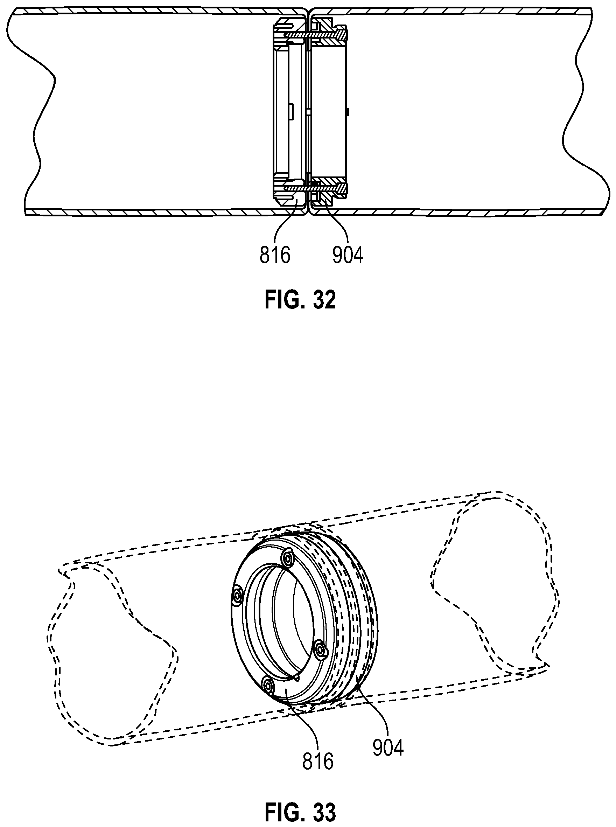

After the compression ring assembly 900 is locked, the connected sections S1 and S2 may be cut along with the head assembly 810 to separate the outer portion 816 (i.e., the distal ring 902) from the inner portion 814. The cutting process may be initiated manually, by pressing the toggle control button 30, or automatically once the locking process is confirmed by the main controller 147, i.e., locking threshold is reached. The cutting process includes advancing the second driver 740 distally, which moves the annular knife 744 disposed concentrically within the proximal ring 904. The annular knife 744 cuts through the connected portions S1 and S2 and thin and/or perforated portion 815 interconnecting the outer portion 816 (i.e., the distal ring 902) and the inner portion 814 of the head assembly 810. This separates the anvil assembly 800 from the outer portion 816 allowing for retraction of the trocar 830 along with inner portion 814 from the locked compression ring assembly 900 that is forming the EEA as shown in FIGS. 32 and 33.

It will be understood that various modifications may be made to the embodiments of the presently disclosed devices. Therefore, the above description should not be construed as limiting, but merely as exemplifications of embodiments. Those skilled in the art will envision other modifications within the scope of the present disclosure.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A surgical device (10) for forming an anastomosis, the device comprising:
an anvil assembly (800) including a head assembly (810) coupled to a rod (820), the head assembly (810) including an outer ring portion coupled to an inner portion via a connection portion;
an annular reload (700) including:
a proximal ring (904) having a plurality of pins (906) movably disposed therein;
a first driver (730) movable distally and configured to engage the plurality of pins (960);
an annular knife (733); and
a second driver movable distally and configured to engage the annular knife;
a first transmission assembly (240) coupled to and configured to move the anvil assembly (800) proximally toward the annular reload (700) to clamp tissue between the anvil assembly (800) and the annular reload (700);
a second transmission assembly (250) coupled to and configured to move the first driver (730) distally to move the plurality of pins (906) through the proximal ring (904), the tissue, and into the outer ring portion to embed the plurality of pins (906) in the outer ring portion; and
a third transmission assembly (260) coupled to and configured to move the second driver and the annular knife (733) through the tissue and the connection portion of the head assembly (810) to separate the outer ring portion from the inner portion;
**characterized in that** the surgical device (10) comprises:
at least one motor configured to move the third transmission assembly;
a sensor configured to measure at least one property of the tissue;
a controller (147) configured to determine whether an anastomosis has been formed or optimal conditions for forming an anastomosis based on the at least one property of the tissue, wherein the controller is further configured to control the at least one motor to move the third transmission assembly based on the determination that the anastomosis has been formed thereby advancing the annular knife through the tissue and the connection portion of the head assembly (810) to separate the outer ring portion from the inner portion.

2. The surgical device (10) according to claim 1, wherein the anvil assembly (800) is formed from a thermoplastic polymer.

3. The surgical device (10) according to claim 1, wherein the at least one motor is configured to move at least one of the first transmission assembly (240), the second transmission assembly (250), or the third transmission assembly (260).

4. The surgical device (10) according to claim 1, wherein the sensor is configured to measure at least one of a mechanical property, optical property, or electrical property of the tissue.

## Patentansprüche

1. Chirurgische Vorrichtung (10) zum Bilden einer Anastomose, wobei die Vorrichtung umfasst:
eine Ambossanordnung (800) mit einer Kopfanordnung (810), die an eine Stange (820) gekoppelt ist, wobei die Kopfanordnung (810) einen äußeren Ringabschnitt aufweist, der über einen Verbindungsabschnitt an einen inneren Abschnitt gekoppelt ist;
eine ringförmige Nachladevorrichtung (700), die aufweist:
einen proximalen Ring (904) mit einer Vielzahl von darin beweglich angeordneten Stiften (906);
einen ersten Treiber (730), der distal beweglich ist und zum Eingriff mit der Vielzahl von Stiften (960) gestaltet ist;
ein Ringmesser (733); und
einen zweiten Treiber, der distal beweglich ist und zum Eingriff mit dem ringförmigen Messer gestaltet ist;
eine erste Getriebeanordnung (240), die an die Ambossanordnung (800) gekoppelt und dafür gestaltet ist, sie proximal in Richtung zu der ringförmigen Nachladevorrichtung (700) zu bewegen, um Gewebe zwischen der Ambossanordnung (800) und der ringförmigen Nachladevorrichtung (700) zu klemmen;
eine zweite Getriebeanordnung (250), die an den ersten Treiber (730) gekoppelt und dafür gestaltet ist, ihn distal zu bewegen, um die Vielzahl von Stiften (906) durch den proximalen Ring (904), das Gewebe und in den äußeren Ringabschnitt zu bewegen, um die Vielzahl von Stiften (906) in den äußeren Ringabschnitt einzubetten; und
eine dritte Getriebeanordnung (260), die an den zweiten Treiber gekoppelt und dafür gestaltet ist, diesen und das ringförmigen Messer (733) durch das Gewebe und den Verbindungsabschnitt der Kopfanordnung (810) zu bewegen, um den äußeren Ringabschnitt von dem inneren Abschnitt zu trennen;
**dadurch gekennzeichnet, dass** die chirurgische Vorrichtung (10) umfasst:
wenigstens einen Motor, gestaltet zum Bewegen der dritten Getriebeanordnung;
einen Sensor, gestaltet zum Messen wenigstens einer Eigenschaft des Gewebes;
eine Steuerung (147), gestaltet zum Bestimmen auf der Grundlage der wenigstens einen eigenschaft des Gewebes, ob eine Anastomose gebildet worden ist oder optimale Bedingungen zum Bilden einer Anastomose vorliegen, wobei die Steuerung ferner dafür gestaltet ist, den wenigstens einen Motor zu steuern, um die dritte Getriebebaugruppe auf der Grundlage der Bestimmung, dass die Anastomose gebildet worden ist, zu bewegen, um das ringförmige Messer durch das Gewebe und den Verbindungsabschnitt der Kopfbaugruppe (810) vorzuschieben, um den äußeren Ringabschnitt von dem inneren Abschnitt zu trennen.

2. Chirurgische Vorrichtung (10) nach Anspruch 1, wobei die Ambossanordnung (800) aus einem thermoplastischen Polymer gebildet ist.

3. Chirurgische Vorrichtung (10) nach Anspruch 1, wobei der wenigstens eine Motor dafür gestaltet ist, wenigstens eine von der ersten Getriebeanordnung (240), der zweiten Getriebeanordnung (250) und der dritten Getriebeanordnung (260) zu bewegen.

4. Chirurgische Vorrichtung (10) nach Anspruch 1, wobei der Sensor dafür gestaltet ist, wenigstens eine von einer mechanischen Eigenschaft, optischen Eigenschaft und elektrischen Eigenschaft des Gewebes zu messen.

## Revendications

1. Dispositif chirurgical (10) pour former une anastomose, le dispositif comprenant :
un ensemble enclume (800) comprenant un ensemble tête (810) couplé à une tige (820), l'ensemble tête (810) comprenant une partie d'anneau externe couplée à une partie interne par l'intermédiaire d'une partie de connexion ;
une recharge annulaire (700) comprenant :
un anneau proximal (904) ayant une pluralité de broches (906) disposées de manière mobile dans celui-ci ;
un premier dispositif d'entraînement (730) mobile de manière distale et configuré pour venir en prise avec la pluralité de broches (960) ;
un couteau annulaire (733) ; et
un second dispositif d'entraînement mobile distalement et configuré pour venir en prise avec le couteau annulaire ;
un premier ensemble de transmission (240) couplé à et configuré pour déplacer l'ensemble enclume (800) de manière proximale vers la recharge annulaire (700) afin de serrer le tissu entre l'ensemble enclume (800) et la recharge annulaire (700) ;
un deuxième ensemble de transmission (250) couplé au premier dispositif d'entraînement (730) et configuré pour le déplacer de manière distale afin de déplacer la pluralité de broches (906) à travers l'anneau proximal (904), le tissu, et dans la partie d'anneau externe afin d'insérer la pluralité de broches (906) dans la partie d'anneau externe ; et
un troisième ensemble de transmission (260) couplé au second dispositif d'entraînement et au couteau annulaire (733) et configuré pour déplacer ce dernier à travers le tissu et la partie de connexion de l'ensemble tête (810) afin de séparer la partie d'anneau externe de la partie interne ;
**caractérisé en ce que** le dispositif chirurgical (10) comprend :
au moins un moteur configuré pour déplacer le troisième ensemble de transmission ;
un capteur configuré pour mesurer au moins une propriété du tissu ;
un dispositif de commande (147) configuré pour déterminer si une anastomose a été formée ou des conditions optimales pour former une anastomose sur la base de l'au moins une propriété du tissu, le dispositif de commande étant en outre configuré pour commander l'au moins un moteur pour déplacer le troisième ensemble de transmission en fonction de la détermination que l'anastomose a été formée, faisant ainsi avancer le couteau annulaire à travers le tissu et la partie de connexion de l'ensemble tête (810) afin de séparer la partie d'anneau externe de la partie interne.

2. Dispositif chirurgical (10) selon la revendication 1, l'ensemble enclume (800) étant formé à partir d'un polymère thermoplastique.

3. Dispositif chirurgical (10) selon la revendication 1, l'au moins un moteur étant configuré pour déplacer au moins l'un parmi le premier ensemble de transmission (240), le deuxième ensemble de transmission (250), ou le troisième ensemble de transmission (260).

4. Dispositif chirurgical (10) selon la revendication 1, le capteur étant configuré pour mesurer au moins l'une d'une propriété mécanique, d'une propriété optique ou d'une propriété électrique du tissu.
